# EUROPEAN PATENT SPECIFICATION

(11) **EP 1 825 838 B1**
(45) Date of publication and mention of the grant of the patent: **23.02.2011**
(21) Application number: 07250783.3
(22) Date of filing: 23.02.2007
(51) Int. Cl.: A61F 5/44

(54) **Urine collection bag with integral anti-reflux valve**
Urinsammeltasche mit integralem Antirefluxventil
Sac de collecte d'urine avec soupape anti-reflux intégral

(30) Priority: 24.02.2006 US 362657
(43) Date of publication of application: 29.08.2007
(73) Proprietor: Tyco Healthcare Group LP, Mansfield, MA 02048 (US)
(72) Inventor: Green, Kurt E, Wrentham, Massachusetts 02093 (US); Salvadori, Lawrence, San Diego, California 92127 (US); Stapleton, Ryan T, Medway, Massachusetts 02053 (US)
(74) Representative: Gray, James

(56) References cited:
- US-A- 3 586 041
- US-A- 3 901 235
- US-A- 3 965 900
- US-A- 3 968 925
- US-A- 4 725 268
- US-A- 5 429 624

## Description

### BACKGROUND

### 1. Technical Field

The present disclosure relates to a urine collection bag for collecting urine from a catheterized patient. More specifically, the present disclosure relates to a urine collection bag for collecting urine from a catheterized patient having an integral anti-reflux valve for substantially preventing fluid from exiting the urine collection bag and flowing back towards the catheterized patient.

### 2. Background Of Related Art

Urine collection bags for collecting urine from a catheterized patient are well known. Typically, urine collection bags are formed from flexible, collapsible front and rear sheets of material which are sealed together at their peripheries to define a fluid reservoir. An inlet tube having a first end in fluid communication with a catheterized patient has a second end positioned to supply urine to an inlet opening of the urine collection bag. An anti-reflux valve can be provided at the inlet to the urine collection bag to prevent fluid from within the urine collection bag from flowing out of the urine collection bag back toward the catheterized patient. Such may occur if the urine collection bag is flipped upside down.

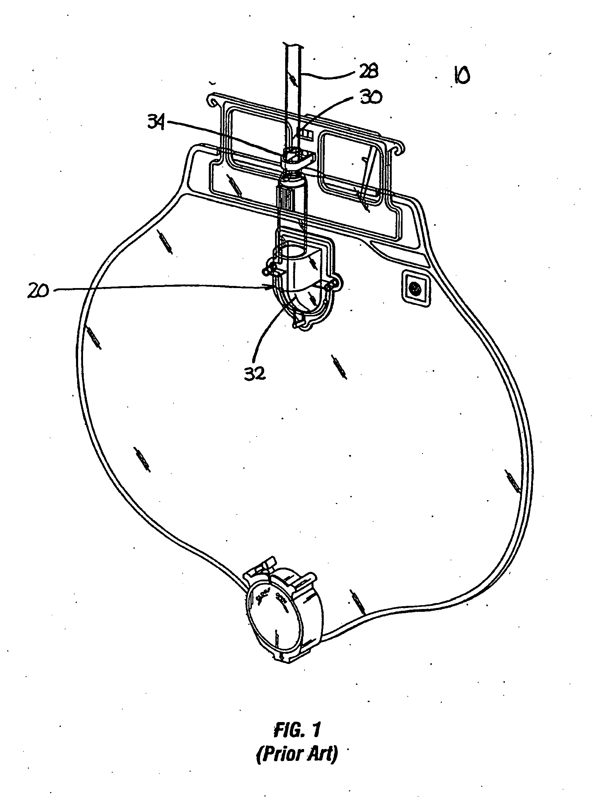

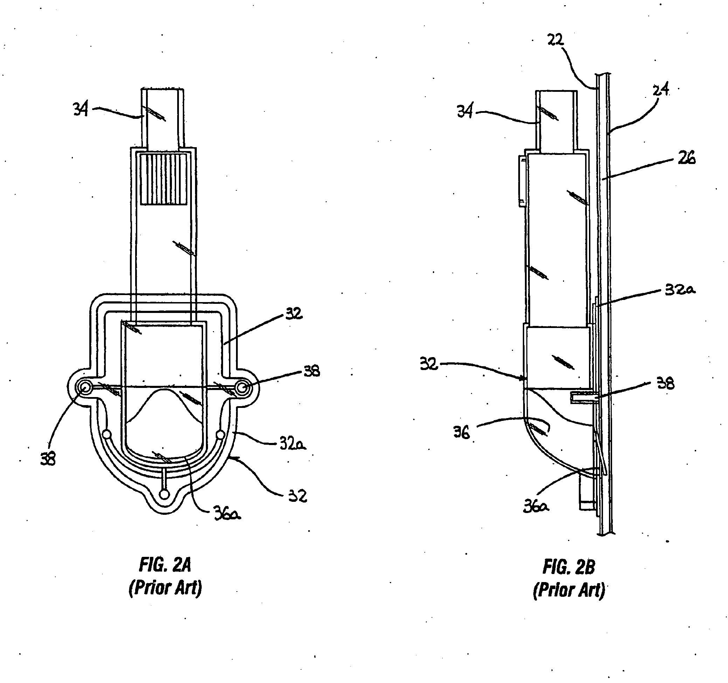

FIGS. 1-3C illustrate one prior art urine collection bag 10 having a known anti-reflux valve 20. Urine collection bag 10 includes first and second flexible sheets of material 22 and 24 (FIG. 2B) which define a collapsible fluid reservoir 26. An inlet tube 28 which is adapted to fluidly communicate with a fluid catheter has a discharge end 30 positioned to drain into anti-reflux valve 20.

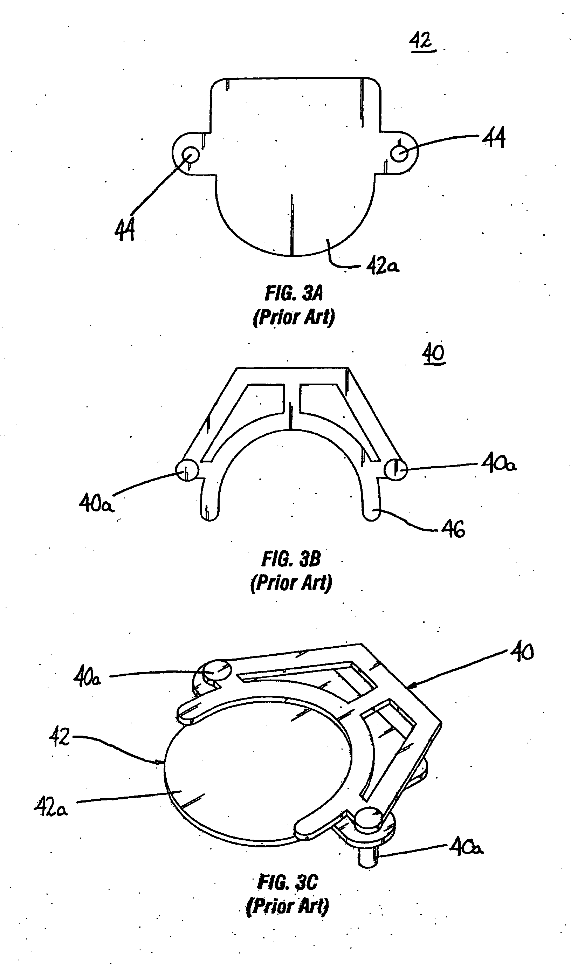

Anti-reflux valve 20 includes a transparent housing 32 having an inlet port 34 dimensioned to receive discharge end 30 of inlet tube 28. Housing 32 defines a fluid receptacle 36 (FIG. 2B) positioned externally of fluid reservoir 26 having an opening 36a into fluid reservoir 26. Housing 32 includes a peripheral portion 32a which is secured or welded to first flexible sheet of material 22. Peripheral portion 32a defines a pair of pin receiving openings 38 positioned on opposite sides of opening 36a. Openings 38 are configured to receive pins 40a of valve support member 40 (FIG. 3B) to secure valve support member 40 adjacent opening 36a. A valve member 42 (FIG. 3C) includes a flexible sheet of material having openings 44 for receiving pins 40a of support member 40. Valve member 42 is supported on pins 40a and is compressed between valve support member 40 and peripheral portion 32a of housing 32 in a position to cover opening 36a.

As illustrated in FIG. 3B, valve support member 40 includes a semi-circular bracket 46 which maintains the upper portion of valve member 42 in a position against peripheral portion 32a of housing 32. However, a lower portion 42a of valve member 42 rests on an outer surface of peripheral portion 32a of housing 32 but is free to move inwardly into reservoir 26 to allow fluid within fluid receptacle 36 to enter reservoir 26. Since valve member 42 overhangs or rests on peripheral portion 32a of housing 32, any fluid from within reservoir 26 contacting valve member 42 tends to seal valve member 42 against peripheral portion 32a of housing 32, thus preventing the flow of fluid from within reservoir 26 into housing 32.

Although the prior art anti-reflux valve described above is quite effective, it would be desirable to provide an anti-reflux valve which is less expensive and more easily manufactured.

US 3901235 discloses an anti-reflux device for urinary collection bags. The bag has a film or sheet flap valve laminate closing against the internal surface of the bag in response to positive pressures within the bag. The valve advantageously permits only a unidirectional flow to occur, thereby preventing any refluxing action of the bag contents from taking place therein. US 3965900 discloses an anti-reflux device for a collection bag having an islet port communicating with the inside of the bag and a generally planar surface surrounding the port on the inside of the bag. The device has a thin flexible valve element having sufficiently large dimensions to cover the inlet port, and means for retaining the valve adjacent the surface on the inside of the bag in a sliding relationship with the inlet port. US 3586041 discloses a flutter check valve for use in a body fluid drainage system. The valve comprises a relatively rigid frame having an opening in communication with the outlet end of the drainage conduit and the flexible film secured to the frame and covering the opening in a position to be pushed away from the opening by fluid from the drainage conduit. US 3968925 discloses an anti-reflux valve mounted at the inlet port to the fluid collection chamber of a fluid collection bag. The valve has a thin flexible valve element which is retained for limited floating movement over the inlet port and which flexes to permit flow of fluid into the collection chamber.

### SUMMARY

In accordance with the present disclosure, a urine collection bag defining a fluid reservoir is disclosed which has the features of claim 1. The anti-reflux valve includes a valve housing defining a fluid receptacle positioned externally of the fluid reservoir. The valve housing includes a peripheral rim which is sealed to a sheet of material defining the urine collection bag such as by welding. An opening is defined by the housing inwardly of the peripheral rim. The valve housing opening is covered by the sheet of material. In one embodiment, a cutout is formed in the sheet of material at a position slightly beneath the housing opening to define a flap. The flap overhangs the peripheral rim of the housing and is movable outwardly into the fluid reservoir to allow fluid flow from the housing receptacle into the fluid reservoir of the urine collection bag. Flow from the reservoir towards the housing receptacle causes the flap to seal against the peripheral rim of the housing and, thus, is substantially prevented. A vent hole interconnecting the fluid reservoir and the housing receptacle can also be provided in the sheet of material.

In one embodiment, the valve housing defines a fluid inlet channel dimensioned to receive one end of an inlet tube. The inlet tube is in fluid communication with a catheter of a catheterized patient.

The urine collection bag can be formed from first and second flexible sheets of material which are sealed at their peripheries to define the fluid reservoir. The first and second sheets of material can be formed of polyvinyl chloride although other flexible materials may be used.

In one embodiment, the valve housing is formed of a substantially transparent material. Alternately, opaque materials may also be used to construct the valve housing.

The urine collection bag can include a discharge valve for regulating drainage of fluid from the fluid reservoir. A vent or vents can also be provided in the urine collection bag.

### BRIEF DESCRIPTION OF THE DRAWINGS

FIG. 1 is a perspective view of a prior art urine collection bag;
FIG. 2A is a front view of a prior art anti-reflux valve of the urine collection bag shown in FIG. 1;
FIG. 2B is a side view of the anti-reflux valve shown in FIG. 2A;
FIG. 3A is a front view of the valve member of the anti-reflux valve shown in FIG. 2B;
FIG. 3B is a front view of the valve support member of the anti-reflux valve shown in FIG. 2B;
FIG. 3C is a side perspective view of the valve support member and valve member of the anti-reflux valve shown in FIG. 2B with the valve member supported on the valve support member;
FIG. 4 is a side perspective view of one embodiment of the presently disclosed urine collection bag including the novel anti-reflux valve;
FIG. 5A is a front view of the anti-reflux valve shown in FIG. 4 with the valve member in the closed position;
FIG. 5B is a side view of the anti-reflux valve shown in FIG. 5A with the valve member in the closed position;
FIG. 6A is a front view of the anti-reflux valve shown in FIG. 5B with the valve member in the open position;
FIG. 6B is a side view of the anti-reflux valve shown in FIG. 6A with the valve member in the open position;
FIG. 7 is a front view of the urine collection bag shown in FIG. 4; and
FIG. 8 is a side view of the urine collection bag shown in FIG. 7.

### DETAILED DESCRIPTION OF EMBODIMENTS

Embodiments of the presently disclosed urine collection bag will now be described in detail with reference to the drawings wherein like reference numerals designate identical or corresponding elements in each of the several views.

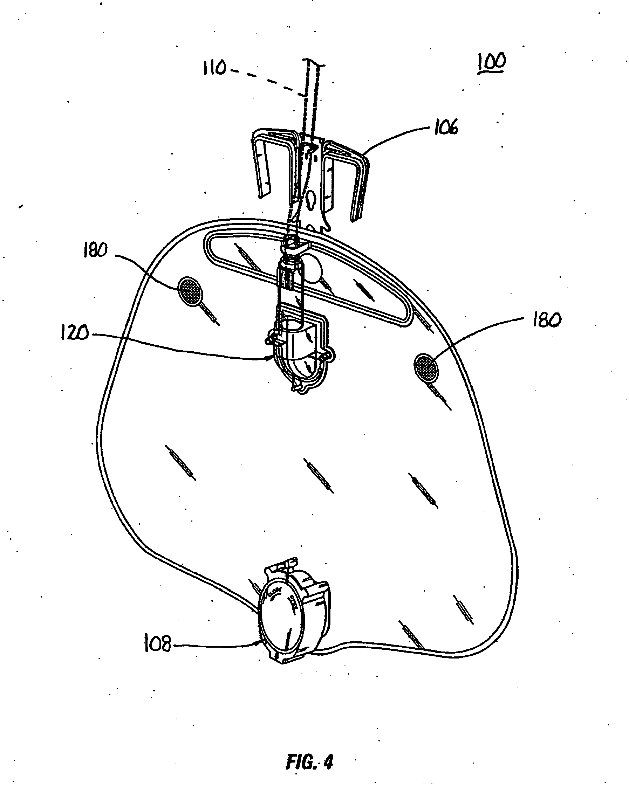

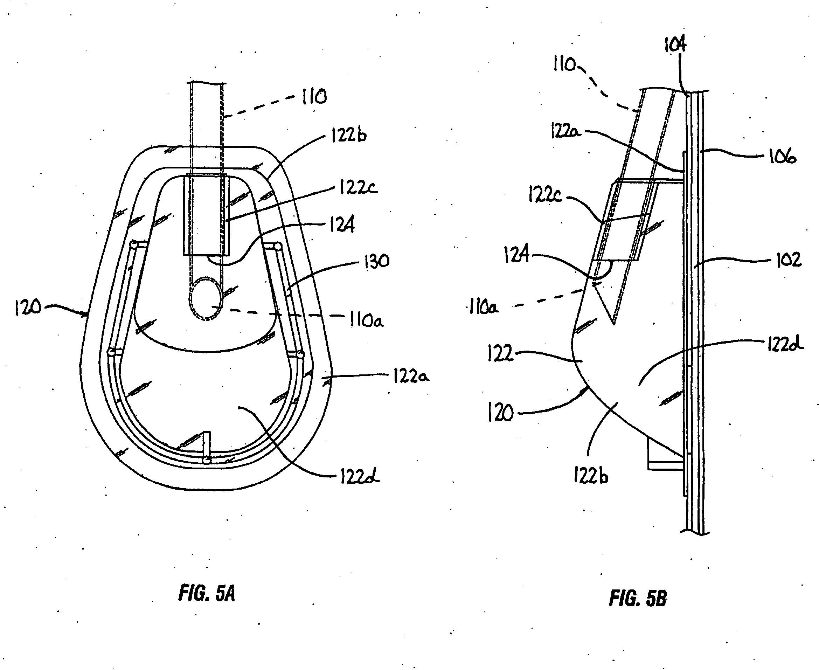

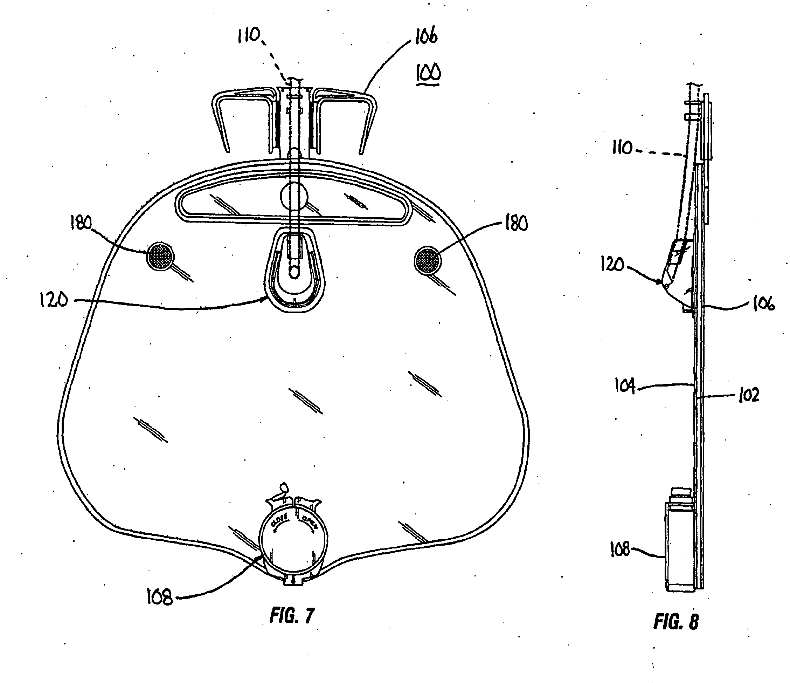

FIGS. 4-8 illustrate one embodiment of the presently disclosed urine collection bag shown generally as 100. Urine collection bag 100 includes at least one sheet of material defining a fluid reservoir 102. In one embodiment, collection bag 100 includes first and second sheets of material 104 and 106 (FIG. 5B) which are sealed at their edges, such as by RF are ultrasonic welding, to define fluid reservoir 102. In a preferred embodiment, urine collection bag 100 is constructed from polyvinyl chloride sheets. Alternately, other flexible or collapsible materials may be used. Collection bag 100 includes an anti-reflux valve 120 which will be discussed in further detail below. Collection bag 100 may also include one or more vents 180, a support or hanger 106 for mounting collection bag 100 on a support structure, e.g., a bed frame, a discharge valve 108 for regulating drainage of fluid from collection bag 102, and/or an inlet tube 110. Inlet tube 110 has one end (not shown) in fluid communication with the catheter of a catheterized patient and a second end 110a positioned to deposit fluid into anti-reflux valve 120.

Referring to FIGS. 5A and 5B, anti-reflux valve 120 includes a housing 122 having an outer rim 122a and a body portion 122b. Housing 122 is preferably formed from a transparent plastic although the use of other materials, transparent or opaque, are also envisioned. Body portion 122b defines a fluid receptacle 122d and a fluid inlet channel 122c dimensioned to receive second end 110a of inlet tube 110 (FIG. 4). Second end 1 10a can be cut at an angle as shown to reduce the likelihood of formation of a meniscus in inlet tube 110. An opening 124 in one end of channel 122c allows fluid to flow from inlet tube 110 into receptacle 122d. The rear side of housing 122 defines an opening 126 (FIG. 5A) which opens into fluid reservoir 102. A stabilizing rib 130 can be provided partially about housing 122 adjacent rim 122a to add rigidity to housing 122.

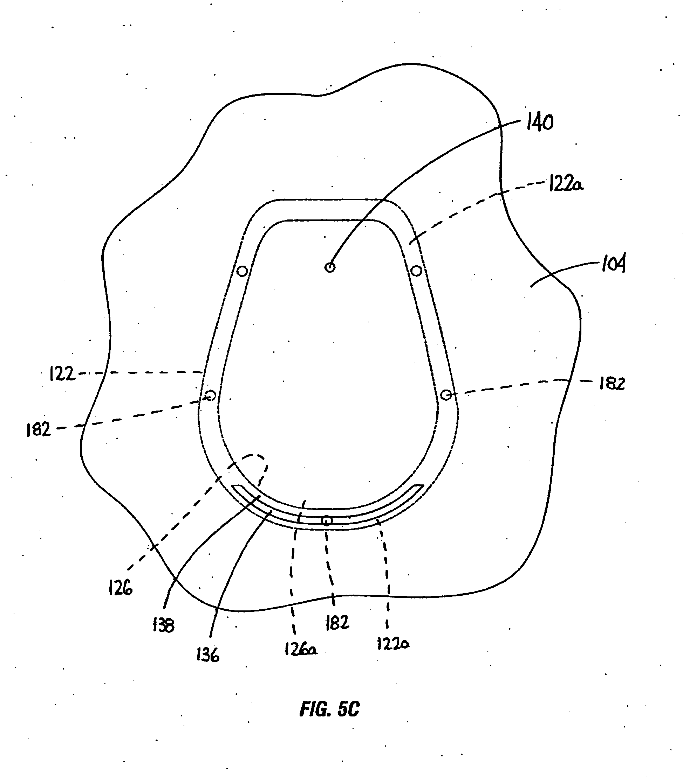

Referring to FIGS. 5B and 5C, rim 122a of housing 122 is secured to first sheet 104 of urine collection bag 100 such as by welding, e.g., RF, ultrasonic, etc. such that sheet 104 covers opening 126 of housing 122. A C-shaped cutout 136 is formed in sheet 104 to define a flap 138 adjacent a bottom edge 126a of opening 126. Flap 138 overhangs rim 122a of housing 122. A small hole 140 is formed in sheet 104. Hole 140 interconnects reservoir 102 and receptacle 122d to allow venting of reservoir 102 as fluid flows from receptacle 122d into reservoir 102. A series of nubs or protrusions 182 is provided about rim 122a to prevent flap 138 from sealing against rim 122a.

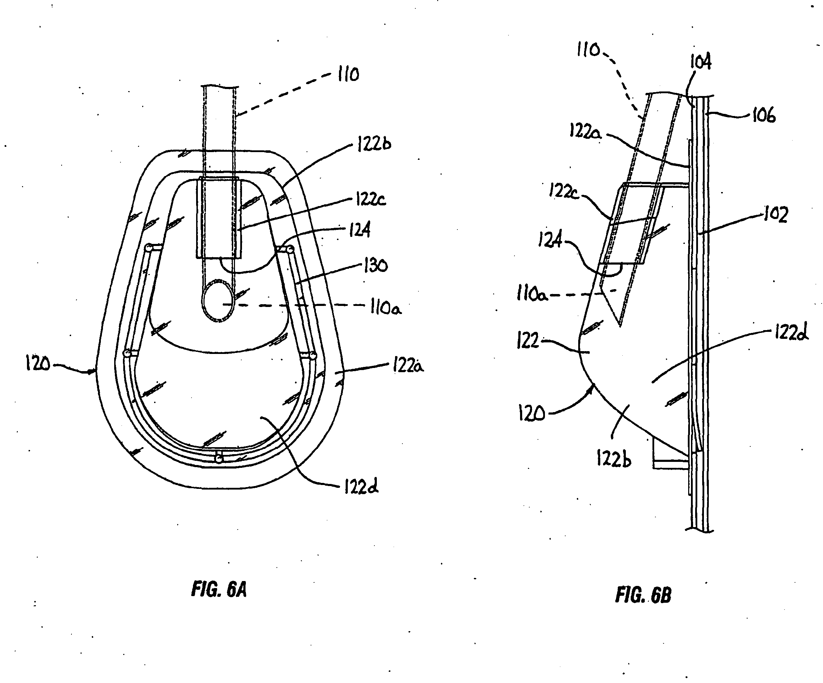

Referring to FIGS. 6A and 6B, when fluid flows through inlet channel 122c into receptacle 122d, the weight of the fluid causes flap 138 to lift off of rim 122a of housing 122 to allow fluid to flow from receptacle 122d into reservoir 102. In contrast, when fluid flows from reservoir 102 back towards anti-reflux valve 120, the weight of the fluid forces flap 138 onto rim 122a of housing 122 to seal opening 126. Because flap 138 is incorporated into sheet 104 of collection bag 100, a less complex, more easily manufacturable and less costly anti-reflux valve is provided.

It will be understood that various modifications may be made to the embodiments disclosed herein. For example, an anti-reflux valve incorporating the flap structure disclosed herein may have an endless number of configurations not illustrated herein. Further, the urine collection bag need not be entirely flexible but can include rigid portions which are secured the flexible sheet defining the flap.

## Claims

1. A urine collection bag (100) comprising:
at least one sheet of flexible material (104,106) defining a fluid reservoir (102); and
an anti-reflux valve (120) including a valve housing (122) defining a fluid receptacle (122d) positioned externally of the fluid reservoir (102), the valve housing (122) including a peripheral rim (122a) which is secured to the at least one sheet of flexible material (104,106) adjacent an inlet opening (136) formed in the at least one sheet of flexible material (104);
wherein the inlet opening (136) in the at least one sheet of material forms a flap (138) in the at least one sheet of flexible material (104), the flap (138) being positioned to abut the peripheral rim (122a) of the housing (122) and substantially seal the inlet opening (136) when fluid flows from the fluid reservoir (102) toward the fluid receptacle, **characterised in that** the peripheral rim (122a) includes a series of protrusions (182) positioned to engage the flap (138) to prevent the flap (138) from sealing against the peripheral rim (122a).

2. A urine collection bag (100) as recited in Claim 1, wherein the valve housing (122) defines a fluid inlet channel (122c) for receiving one end of an inlet tube (110).

3. A urine collection bag (100) as recited in Claim 1, wherein the at least one sheet of flexible material includes first and second sheets of material (104,106) secured together at their peripheries to define the fluid reservoir (102).

4. A urine collection bag (100) as recited in Claim 3, wherein the first and second sheets of material (104,106) are formed from polyvinyl chloride.

5. A urine collection bag (100) as recited in Claim 1, wherein the valve housing (122) is substantially transparent.

6. A urine collection bag (100) as recited in Claim 1, wherein the urine collection bag (100) includes a discharge valve (108) for regulating drainage of the fluid reservoir (102).

7. A urine collection bag (100) according to Claim 3, wherein the peripheral rim (122a) of the valve housing (122) is welded to the first sheet of flexible material (104).

8. A urine collection bag (100) according to Claim 7, wherein the valve housing (122) defines an opening inwardly of the peripheral rim (122a).

9. A urine collection bag (100) according to Claim 8, wherein the opening is covered by the first sheet of flexible material (104).

10. A urine collection bag (100) according to Claim 9, wherein the inlet opening is formed in the first sheet of flexible material (104) at a position below the valve housing opening (122c).

11. A urine collection bag (100) according to Claim 3, further including a vent hole (180) formed in the first sheet of material (104), the vent hole (180) interconnecting the fluid reservoir (102) and the fluid receptacle (122d).

## Patentansprüche

1. Urinsammeltasche (100), die Folgendes umfasst:
zumindest ein Flächenstück aus einem flexiblen Material (104, 106), das ein Flüssigkeitsreservoir (102) definiert; und ein Antirefluxventil (120) mit einem Ventilgehäuse (122), das einen außerhalb des Flüssigkeitsreservoirs (102) angeordneten Flüssigkeitsaufnahmebehälter (122d) definiert, wobei das Ventilgehäuse (122) einen Umfangsrand (122a) aufweist, der neben einer Einlassöffnung (136) in dem zumindest einen Flächenstück aus flexiblem Material (104) an dem zumindest einen Flächenstück aus flexiblem Material (104, 106) befestigt ist; worin die Einlassöffnung (136) in dem zumindest einen Flächenstück aus flexiblem Material eine Klappe (138) in dem zumindest einen Flächenstück aus flexiblem Material (104) bildet, wobei die Klappe (138) so angeordnet ist, dass sie an den peripheren Rand (122a) des Gehäuses (122) anstößt und die Einlassöffnung (136) im Wesentlichen abdichtet, wenn Flüssigkeit aus dem Flüssigkeitsreservoir (102) zum Flüssigkeitsaufnahmebehälter fließt, **dadurch gekennzeichnet, dass** der Umfangsrand (122a) eine Reihe von Vorsprüngen (182) aufweist, die so angeordnet sind, dass sie in die Klappe (138) eingreifen, um zu verhindern, dass die Klappe (138) den Umfangsrand (122a) abdichtet.

2. Urinsammeltasche (100) nach Anspruch 1, worin das Ventilgehäuse (122) einen Flüssigkeitseinlasskanal (122c) zur Aufnahme eines Endes eines Einlassrohrs (110) definiert.

3. Urinsammeltasche (100) nach Anspruch 1, worin das zumindest eine Flächenstück aus flexiblem Material erste und zweite Materialflächenstücke (104, 106) aufweist, die an ihrem Umfang zur Definition des Flüssigkeitsreservoirs (102) befestigt sind.

4. Urinsammeltasche (100) nach Anspruch 3, worin die ersten und zweiten Materialflächenstücke (104, 106) aus Polyvinylchlorid bestehen.

5. Urinsammeltasche (100) nach Anspruch 1, worin das Ventilgehäuse (122) im Wesentlichen transparent ist.

6. Urinsammeltasche (100) nach Anspruch 1, worin die Urinsammeltasche (100) ein Entleerungsventil (108) zur Regulierung der Drainage des Flüssigkeitsreservoirs (102) aufweist.

7. Urinsammeltasche (100) nach Anspruch 3, worin der Umfangsrand (122a) des Ventilgehäuses (122) an dem ersten Flächenstück aus flexiblem Material (104) festgeschweißt ist.

8. Urinsammeltasche (100) nach Anspruch 7, worin das Ventilgehäuse (122) am Umfangsrand (122a) einwärts eine Öffnung definiert.

9. Urinsammeltasche (100) nach Anspruch 8, worin die Öffnung von dem ersten Flächenstück aus flexiblem Material (104) abgedeckt ist.

10. Urinsammeltasche (100) nach Anspruch 9, worin die Einlassöffnung im ersten Flächenstück aus flexiblem Material (104) an einer Stelle unter der Ventilgehäuseöffnung (122c) gebildet ist.

11. Urinsammeltasche (100) nach Anspruch 3, die ferner ein Entlüftungsloch (180) im ersten Flächenstück aus flexiblem Material (104) aufweist, wobei das Entlüftungsloch (180) das Flüssigkeitsreservoir (102) mit dem Flüssigkeitsaufnahmebehälter (122d) verbindet.

## Revendications

1. Poche de collecte d'urine (100) comprenant :
au moins une feuille de matériau souple (104, 106) définissant un réservoir de fluide (102) ; et
une valve anti-reflux (120) comprenant un logement de valve (122) définissant un réceptacle de fluide (122d) positionné extérieurement au réservoir de fluide (102), le logement de valve (122) comprenant une bordure périphérique (122a) qui est fixée à l'au moins une feuille de matériau souple (104, 106) en un point adjacent à une ouverture d'entrée (136) formée dans l'au moins une feuille de matériau souple (104) ;
où l'ouverture d'entrée (136) dans l'au moins une feuille de matériau forme un rabat (138) dans l'au moins une feuille de matériau souple (104), le rabat (138) étant positionné de manière à buter contre la bordure périphérique (122a) du logement (122) et sensiblement obturer l'ouverture d'entrée (136) lorsque le fluide s'écoule depuis le réservoir de fluide (102) vers le réceptacle de fluide, **caractérisée en ce que** la bordure périphérique (122a) comprend une série de saillies (182) positionnées pour engager le rabat (138) afin d'empêcher que le rabat (138) se soude contre la bordure périphérique (122a).

2. Poche de collecte d'urine (100) selon la revendication 1, dans laquelle le logement de valve (122) définit un canal d'entrée de fluide (122c) destiné à recevoir une extrémité d'un tube d'entrée (110).

3. Poche de collecte d'urine (100) selon la revendication 1, dans laquelle l'au moins une feuille de matériau souple comprend des première et deuxième feuilles de matériau (104, 106) fixées l'une à l'autre à leur périphérie pour définir le réservoir de fluide (102).

4. Poche de collecte d'urine (100) selon la revendication 3, dans laquelle les première et deuxième feuilles de matériau (104, 106) sont formées de polychlorure de vinyle.

5. Poche de collecte d'urine (100) selon la revendication 1, dans laquelle le logement de valve (122) est sensiblement transparent.

6. Poche de collecte d'urine (100) selon la revendication 1, la poche de collecte d'urine (100) comprenant une valve de décharge (108) pour réguler l'évacuation du réservoir de fluide (102).

7. Poche de collecte d'urine (100) selon la revendication 3, dans laquelle la bordure périphérique (122a) du logement de valve (122) est soudée à la première feuille de matériau souple (104).

8. Poche de collecte d'urine (100) selon la revendication 7, dans laquelle le logement de valve (122) définit une ouverture vers l'intérieur de la bordure périphérique (122a).

9. Poche de collecte d'urine (100) selon la revendication 8, dans laquelle l'ouverture est recouverte par la première feuille de matériau souple (104).

10. Poche de collecte d'urine (100) selon la revendication 9, dans laquelle l'ouverture d'entrée est formée dans la première feuille de matériau souple (104) en une position au-dessous de l'ouverture (122c) de logement de valve.

11. Poche de collecte d'urine (100) selon la revendication 3, comprenant, en outre, un évent (180) formé dans la première feuille de matériau (104), l'évent (180) reliant le réservoir de fluide (102) et le réceptacle de fluide (122d) l'un à l'autre.
